(19) Europäisches Patentamt
European Patent Office
Office européen des brevets

(11) EP 3 438 193 A1

(12) EUROPEAN PATENT APPLICATION
published in accordance with Art. 153(4) EPC

(43) Date of publication:
**06.02.2019 Bulletin 2019/06**

(21) Application number: **17774585.8**

(22) Date of filing: **22.03.2017**

(51) Int Cl.:
***C08L 33/00*** *(2006.01)* ***C08K 5/20*** *(2006.01)*
***C07C 233/36*** *(2006.01)* ***C07C 233/78*** *(2006.01)*
***C07C 235/50*** *(2006.01)* ***C07C 255/57*** *(2006.01)*

(86) International application number:
**PCT/JP2017/011459**

(87) International publication number:
**WO 2017/170044 (05.10.2017 Gazette 2017/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.03.2016 JP 2016068280**

(71) Applicant: **Zeon Corporation**
**Tokyo 100-8246 (JP)**

(72) Inventors:
- **SUGAWARA, Mitsuru**
  **Tokyo 100-8246 (JP)**
- **KIRIKI, Satoshi**
  **Tokyo 100-8246 (JP)**
- **SAKAMOTO, Kei**
  **Tokyo 100-8246 (JP)**
- **EJIRI, Kazuhiro**
  **Tokyo 100-8246 (JP)**

(74) Representative: **Maiwald Patent- und Rechtsanwaltsgesellschaft mbH**
**Elisenhof**
**Elisenstraße 3**
**80335 München (DE)**

# (54) ACRYLIC POLYMER COMPOSITION

(57) Provided is an acrylic polymer composition comprising an acrylic polymer and a polyfunctional organic compound represented by the following general formula (1):

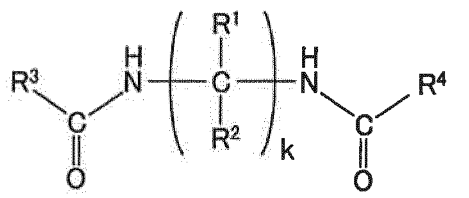

(1)

(in the general formula (1), $R^1$ and $R^2$ are each independently a hydrogen atom or an alkyl group having 1 to 20 carbon atoms, $R^3$ and $R^4$ are each independently a hydrogen atom, a substituted or unsubstituted alkyl group having 1 to 20 carbon atoms, or a substituted or unsubstituted aryl group having 6 to 10 carbon atoms, and k is an integer of 1 or larger).

EP 3 438 193 A1

## Description

### TECHNICAL FIELD

The disclosure relates to an acrylic polymer composition, and in particular, relates to an acrylic polymer composition effectively prevented from being thermally degraded under heating.

### BACKGROUND ART

With developments in petrochemistry, polymers constituted by organic compounds have contributed in various forms such as plastics, rubbers, fibers, and films to the evolution of humans. These polymers are used in various environments according to purposes and as such, have each been improved such that they can be used for a long period by imparting durability thereto in expected environments. For example, plastics for outdoor use have been developed as products provided with ultraviolet-resistant performance, and rubbers functioning even in severe cold areas have been developed as products provided with cold-resistant performance.

Meanwhile, internal combustions typified by engines, which have been used in increased amounts with industrial developments, require lubricating oils and also generate a great deal of heat, and therefore, polymers for use therein are required to have resistance to oils or high temperatures. Particularly, polymers for automobile engines or their surroundings are required to have properties of being able to maintain flexibility for a long time and not causing defects such as cracks, even when exposed to oils or high temperatures. In response to such requirements, various oil-resistant and/or heat-resistant rubbers have been developed. Among others, acrylic polymers are widely used as polymers having rubber elasticity and having excellent oil resistance, heat-resistant properties, and flexibility and as members such as seals, gaskets, packings, and hoses for automobile engines or their surroundings. Their oil resistance and heat resistance are further enhanced by devising cross-linked structures, antioxidants, or compounding agents according to required properties. For example, Patent Document 1 discloses an antioxidant which improves heat resistance. However, such an antioxidant alone cannot suppress decrease in molecular weights of polymers under heat and is therefore insufficient for responding to further requirements for heat resistance at 190°C or higher.

Meanwhile, for biopolymers, etc., in order to cope with decrease in molecular weight caused by molecular chain scission, a technique of self-repairing the molecular chain using a repairing agent coexisting therewith is widely exploited. This technique, however, is often used *in vivo* and therefore, is not used in high-temperature regions. Patent Document 2 discloses a technique of self-repairing the molecular chain scission of polyester resin through the recombination reaction of a repairing agent by using 1,4-butylene glycol, bis(2-hydroxyethyl) terephthalate, or dimethyl phthalate as the repairing agent, as means of suppressing decrease in molecular weights of polymers in high-temperature regions. This technique of Patent Document 2 prevents decrease in molecular weight by self-repairing through the recombination reaction of the repairing agent and thereby suppresses thermal degradation under heating. This document, however, merely discloses a degradation suppressive effect at 150°C and does not report a technique of suppressing degradation in higher-temperature regions of 190°C or higher.

### RELATED ART

### PATENT DOCUMENTS

Patent Document 1: Japanese Patent No. 5682575
Patent Document 2: Japanese Patent Laid-Open No. 2005-23166

### SUMMARY OF THE INVENTION

### TECHNICAL PROBLEM

The present invention has been made in light of the actual situation described above, and an object of the present invention is to provide an acrylic polymer composition effectively prevented from being thermally degraded under heating even in a high-temperature environment (e.g., in an environment of 190°C or higher) .

### SOLUTION TO PROBLEM

The present inventors have conducted diligent studies to attain the object and consequently completed the

present invention by finding that the object can be attained by an acrylic polymer composition prepared by blending an acrylic polymer with a specific polyfunctional organic compound having two amide groups.

Specifically, one aspect of the present invention provides an acrylic polymer composition comprising an acrylic polymer and a polyfunctional organic compound represented by the following general formula (1):

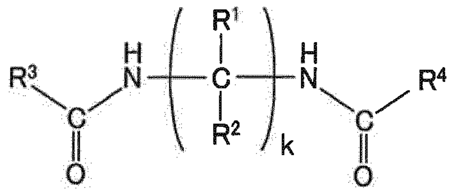

(1)

(in the general formula (1), $R^1$ and $R^2$ are each independently a hydrogen atom or an alkyl group having 1 to 20 carbon atoms, $R^3$ and $R^4$ are each independently a hydrogen atom, a substituted or unsubstituted alkyl group having 1 to 20 carbon atoms, or a substituted or unsubstituted aryl group having 6 to 10 carbon atoms, and k is an integer of 1 or larger).

In one aspect of the present invention, preferably, the polyfunctional organic compound is a compound represented by the following general formula (2):

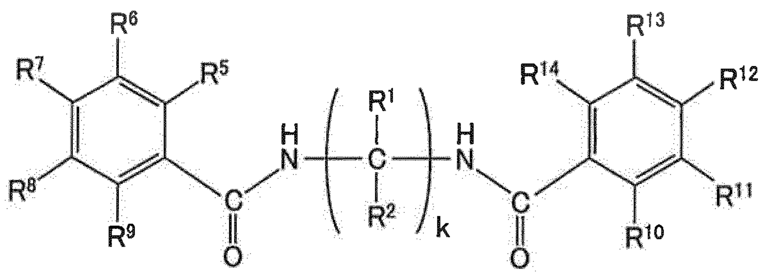

(2)

(in the general formula (2), $R^1$, $R^2$, and k are as defined in the general formula (1), and $R^5$ to $R^{14}$ are each independently a hydrogen atom, a halogen atom, an alkoxy group having 1 to 10 carbon atoms, a nitro group, a cyano group, a substituted or unsubstituted aryl group having 6 to 10 carbon atoms, or an alkyl group having 1 to 20 carbon atoms).

In one aspect of the present invention, preferably, in the general formula (1) or the general formula (2), k is 6.

In one aspect of the present invention, preferably, the polyfunctional organic compound has a half-life of 5 hours or longer at 190°C.

In one aspect of the present invention, preferably, a content ratio of the polyfunctional organic compound is an amount of 0.002 or more in terms of the number of equivalents of the amide group contained in the polyfunctional organic compound with respect to an ester group contained in the acrylic polymer.

In one aspect of the present invention, preferably, a content ratio of the polyfunctional organic compound is 0.4 parts by weight or more in terms of a weight ratio per 100 parts by weight of the acrylic polymer.

One aspect of the present invention also provides a cross-linked product prepared by cross-linking the acrylic polymer composition of one aspect of the present invention described above.

One aspect of the present invention further provides a cross-linkable acrylic polymer composition comprising an acrylic polymer, a polyfunctional organic compound represented by the general formula (1), and a cross-linking agent.

## ADVANTAGEOUS EFFECTS

One aspect of the present invention can provide an acrylic polymer composition effectively prevented from being thermally degraded under heating even in a high-temperature environment (e.g., in an environment of 190°C or higher) .

## DESCRIPTION OF EMBODIMENTS

The acrylic polymer composition of one embodiment of the present invention comprises an acrylic polymer and a polyfunctional organic compound represented by the general formula (1) mentioned later.

<Acrylic polymer>

The acrylic polymer used in one embodiment of the present invention is not particularly limited as long as the acrylic polymer contains a (meth)acrylic acid ester monomer [which means an acrylic acid ester monomer and/or a methacrylic acid ester monomer; the same holds true for methyl (meth)acrylate, etc. described below] unit as a main component (which refers to, in the present disclosure, a component that occupies 50% by weight or more of all monomer units in the polymer) in the molecule.

Examples of the (meth)acrylic acid ester monomer that forms the (meth)acrylic acid ester monomer unit as a main component in the acrylic polymer used in one embodiment of the present invention can include, but are not particularly limited to, (meth)acrylic acid alkyl ester monomers, (meth)acrylic acid alkoxyalkyl ester monomers, and the like.

The (meth)acrylic acid alkyl ester monomer is not particularly limited and is preferably an ester of an alkanol having 1 to 8 carbon atoms and (meth)acrylic acid. Specific examples thereof include methyl (meth)acrylate, ethyl (meth)acrylate, n-propyl (meth)acrylate, isopropyl (meth)acrylate, n-butyl (meth)acrylate, isobutyl (meth)acrylate, n-hexyl (meth)acrylate, 2-ethylhexyl (meth)acrylate, cyclohexyl (meth)acrylate, and the like. Among them, ethyl (meth)acrylate and n-butyl (meth)acrylate are preferred, and ethyl acrylate and n-butyl acrylate are particularly preferred. These (meth) acrylic acid alkyl ester monomers can be used alone or in combination of two or more thereof.

The (meth)acrylic acid alkoxyalkyl ester monomer is not particularly limited and is preferably an ester of an alkoxyalkyl alcohol having 2 to 8 carbon atoms and (meth)acrylic acid. Specific examples thereof include methoxymethyl (meth)acrylate, ethoxymethyl (meth)acrylate, 2-methoxyethyl (meth)acrylate, 2-ethoxyethyl (meth)acrylate, 2-propoxyethyl (meth)acrylate, 2-butoxyethyl (meth)acrylate, 3-methoxypropyl (meth) acrylate, 4-methoxybutyl (meth) acrylate, and the like. Among them, 2-ethoxyethyl (meth)acrylate and 2-methoxyethyl (meth)acrylate are preferred, and 2-ethoxyethyl acrylate and 2-methoxyethyl acrylate are particularly preferred. These (meth)acrylic acid alkoxyalkyl ester monomers can be used alone or in combination of two or more thereof.

The content of the (meth)acrylic acid ester monomer unit in the acrylic polymer used in one embodiment of the present invention is 50 to 100% by weight, preferably 50 to 99.9% by weight, more preferably 60 to 99.5% by weight, further preferably 70 to 99.5% by weight, particularly preferably 70 to 99% by weight. If the content of the (meth)acrylic acid ester monomer unit is too small, weather resistance, heat resistance, and oil resistance might be reduced.

In one embodiment of the present invention, the (meth)acrylic acid ester monomer unit preferably consists of 30 to 100% by weight of the (meth)acrylic acid alkyl ester monomer unit and 70 to 0% by weight of the (meth)acrylic acid alkoxyalkyl ester monomer unit.

Also, the acrylic polymer used in one embodiment of the present invention may contain a cross-linkable monomer unit in addition to the (meth)acrylic acid ester monomer unit.

Examples of the cross-linkable monomer that forms the cross-linkable monomer unit include, but are not particularly limited to: $\alpha,\beta$-ethylenic unsaturated carboxylic acid monomers; monomers having an epoxy group; monomers having a halogen atom; diene monomers; and the like.

Examples of the $\alpha,\beta$-ethylenic unsaturated carboxylic acid monomer include, but are not particularly limited to, $\alpha,\beta$-ethylenic unsaturated monocarboxylic acids having 3 to 12 carbon atoms, $\alpha,\beta$-ethylenic unsaturated dicarboxylic acids having 4 to 12 carbon atoms, monoesters of $\alpha,\beta$-ethylenic unsaturated dicarboxylic acids having 4 to 12 carbon atoms and alkanols having 1 to 8 carbon atoms, and the like.

Specific examples of the $\alpha,\beta$-ethylenic unsaturated monocarboxylic acid having 3 to 12 carbon atoms include acrylic acid, methacrylic acid, $\alpha$-ethylacrylic acid, crotonic acid, cinnamic acid, and the like.

Specific examples of the $\alpha,\beta$-ethylenic unsaturated dicarboxylic acid having 4 to 12 carbon atoms include: butenedioic acids such as fumaric acid and maleic acid; itaconic acid; citraconic acid; chloromaleic acid; and the like.

Specific examples of the monoester of an $\alpha,\beta$-ethylenic unsaturated dicarboxylic acid having 4 to 12 carbon atoms and an alkanol having 1 to 8 carbon atoms include: butenedioic acid mono-linear alkyl esters such as monomethyl fumarate, monoethyl fumarate, mono-n-butyl fumarate, monomethyl maleate, monoethyl maleate, and mono-n-butyl maleate; butenedioic acid monoesters having an alicyclic structure, such as monocyclopentyl fumarate, monocyclohexyl fumarate, monocyclohexenyl fumarate, monocyclopentyl maleate, monocyclohexyl maleate, and monocyclohexenyl maleate; itaconic acid monoesters such as monomethyl itaconate, monoethyl itaconate, mono-n-butyl itaconate, and monocyclohexyl itaconate; and the like.

Among them, a butenedioic acid mono-linear alkyl ester and a butenedioic acid monoester having an alicyclic structure are preferred, mono-n-butyl fumarate, mono-n-butyl maleate, monocyclohexyl fumarate, and monocyclohexyl maleate are more preferred, and mono-n-butyl fumarate is further preferred. These $\alpha,\beta$-ethylenic unsaturated carboxylic acid monomers can be used alone or in combination of two or more thereof. Among the monomers described above, the dicarboxylic acid also includes forms present as an anhydride.

Examples of the monomer having an epoxy group include, but are not particularly limited to, epoxy group-containing (meth)acrylic acid esters, epoxy group-containing ethers, and the like.

Specific examples of the epoxy group-containing (meth)acrylic acid ester include glycidyl (meth)acrylate.

Specific examples of the epoxy group-containing ether include allyl glycidyl ether, vinyl glycidyl ether, and the like. Among them, glycidyl methacrylate and allyl glycidyl ether are preferred. These monomers having an epoxy group can be used alone or in combination of two or more thereof.

Examples of the monomer having a halogen atom include, but are not particularly limited to, unsaturated alcohol esters of halogen-containing saturated carboxylic acids, (meth)acrylic acid haloalkyl esters, (meth)acrylic acid haloacyloxyalkyl esters, (meth)acrylic acid (haloacetylcarbamoyloxy)alkyl esters, halogen-containing unsaturated ethers, halogen-containing unsaturated ketones, halomethyl group-containing aromatic vinyl compounds, halogen-containing unsaturated amides, haloacetyl group-containing unsaturated monomers, and the like.

Specific examples of the unsaturated alcohol ester of a halogen-containing saturated carboxylic acid include vinyl chloroacetate, vinyl 2-chloropropionate, allyl chloroacetate, and the like.

Specific examples of the (meth)acrylic acid haloalkyl ester include chloromethyl (meth)acrylate, 1-chloroethyl (meth)acrylate, 2-chloroethyl (meth)acrylate, 1,2-dichloroethyl (meth)acrylate, 2-chloropropyl (meth)acrylate, 3-chloropropyl (meth)acrylate, 2,3-dichloropropyl (meth)acrylate, and the like.

Specific examples of the (meth)acrylic acid haloacyloxyalkyl ester include 2-(chloroacetoxy)ethyl (meth)acrylate, 2-(chloroacetoxy)propyl (meth)acrylate, 3-(chloroacetoxy)propyl (meth)acrylate, 3-(hydroxychloroacetoxy)propyl (meth)acrylate, and the like.

Specific examples of the (meth)acrylic acid (haloacetylcarbamoyloxy)alkyl ester include 2-(chloroacetylcarbamoyloxy)ethyl (meth)acrylate, 3-(chloroacetylcarbamoyloxy)propyl (meth)acrylate, and the like.

Specific examples of the halogen-containing unsaturated ether include chloromethyl vinyl ether, 2-chloroethyl vinyl ether, 3-chloropropyl vinyl ether, 2-chloroethyl allyl ether, 3-chloropropyl allyl ether, and the like.

Specific examples of the halogen-containing unsaturated ketone include 2-chloroethyl vinyl ketone, 3-chloropropyl vinyl ketone, 2-chloroethyl allyl ketone, and the like.

Specific examples of the halomethyl group-containing aromatic vinyl compound include p-chloromethylstyrene, m-chloromethylstyrene, o-chloromethylstyrene, p-chloromethyl-α-methylstyrene, and the like.

Specific examples of the halogen-containing unsaturated amide include N-chloromethyl(meth)acrylamide, and the like.

Specific examples of the haloacetyl group-containing unsaturated monomer include 3-(hydroxychloroacetoxy)propyl allyl ether, p-vinylbenzyl chloroacetic acid ester, and the like.

Among them, an unsaturated alcohol ester of a halogen-containing saturated carboxylic acid and a halogen-containing unsaturated ether are preferred, vinyl chloroacetate and 2-chloroethyl vinyl ether are more preferred, and vinyl chloroacetate is further preferred. These monomers having a halogen atom can be used alone or in combination of two or more thereof.

Examples of the diene monomer include conjugated diene monomers and non-conjugated diene monomers.

Specific examples of the conjugated diene monomer can include 1,3-butadiene, isoprene, piperylene, and the like.

Specific examples of the non-conjugated diene monomer can include ethylidene norbornene, dicyclopentadiene, dicyclopentadienyl (meth)acrylate, 2-dicyclopentadienyl ethyl (meth)acrylate, and the like.

The cross-linkable monomers mentioned above can be used alone or in combination of two or more thereof.

When the acrylic polymer used in one embodiment of the present invention contains the cross-linkable monomer unit, the content of the cross-linkable monomer unit in the acrylic polymer is preferably 0.1 to 10% by weight, more preferably 0.5 to 7% by weight, further preferably 1 to 5% by weight.

Also, the acrylic polymer used in one embodiment of the present invention may have, if necessary, a unit of an additional monomer copolymerizable with the (meth)acrylic acid ester monomer.

Examples of the copolymerizable additional monomer include, but are not particularly limited to, aromatic vinyl monomers, α,β-ethylenic unsaturated nitrile monomers, monomers having two or more acryloyloxy groups (hereinafter, also referred to as "polyfunctional acrylic monomers"), olefinic monomers, vinyl ether compounds, and the like.

Specific examples of the aromatic vinyl monomer include styrene, α-methylstyrene, divinylbenzene, and the like.

Specific examples of the α,β-ethylenic unsaturated nitrile monomer include acrylonitrile, methacrylonitrile, and the like.

Specific examples of the polyfunctional acrylic monomer include ethylene glycol di(meth)acrylate, propylene glycol di(meth)acrylate, and the like.

Specific examples of the olefinic monomer include ethylene, propylene, 1-butene, 1-octene, and the like.

Specific examples of the vinyl ether compound include vinyl acetate, ethyl vinyl ether, n-butyl vinyl ether, and the like.

Among them, styrene, acrylonitrile, methacrylonitrile, ethylene and vinyl acetate are preferred, and acrylonitrile, methacrylonitrile, ethylene and vinyl acetate are more preferred.

These copolymerizable additional monomers can be used alone or in combination of two or more thereof.

The content of the unit of the additional monomer in the acrylic polymer is preferably 0 to 50% by weight, more preferably 0 to 49.9% by weight, further preferably 0 to 39.5% by weight, particularly preferably 0 to 29.5% by weight.

The acrylic polymer used in one embodiment of the present invention can be obtained by polymerizing the monomers described above. Any of emulsion polymerization, suspension polymerization, bulk polymerization, and solution polymerization methods can be used as a form of polymerization reaction. An emulsion polymerization method under normal pressure which is generally used as a method for producing a heretofore known acrylic polymer is preferred from the viewpoint of easy control of the polymerization reaction, etc.

The emulsion polymerization may be of a batch, semi-batch, or continuous type. The polymerization is usually performed in the temperature range of 0 to 70°C, preferably 5 to 50°C.

The peak top molecular weight (Mp) of the acrylic polymer used in one embodiment of the present invention is not particularly limited and is preferably 20,000 to 2,000,000, more preferably 40,000 to 1,600,000, further preferably 60,000 to 1,400,000. The peak top molecular weight (Mp) of the acrylic polymer can be measured, for example, as a polystyrene-based value by gel permeation chromatography.

In terms of a weight-average molecular weight (Mw), the weight-average molecular weight of the acrylic polymer used in one embodiment of the present invention is preferably 50,000 to 5,000,000, more preferably 100,000 to 4,000,000, further preferably 150,000 to 3,500,000.

The Mooney viscosity ($ML_{1+4}$, 100°C) (polymer Mooney) of the thus-produced acrylic polymer used in one embodiment of the present invention is preferably 10 to 80, more preferably 20 to 70, further preferably 25 to 60.

<Polyfunctional organic compound>

The acrylic polymer composition of one embodiment of the present invention is prepared by blending the acrylic polymer mentioned above with a polyfunctional organic compound represented by the following general formula (1):

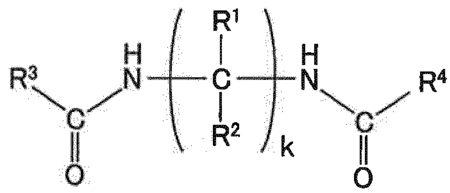

(1)

(in the general formula (1), $R^1$ and $R^2$ are each independently a hydrogen atom or an alkyl group having 1 to 20 carbon atoms, $R^3$ and $R^4$ are each independently a hydrogen atom, a substituted or unsubstituted alkyl group having 1 to 20 carbon atoms, or a substituted or unsubstituted aryl group having 6 to 10 carbon atoms, and k is an integer of 1 or larger).

When an ordinary acrylic polymer contained in the acrylic polymer composition of one embodiment of the present invention, or the like is heated to 190°C or higher, the molecular chain scission of the acrylic polymer usually occurs. This decreases its molecular weight so that the acrylic polymer is disadvantageously degraded under heating. By contrast, according to one embodiment of the present invention, the acrylic polymer is blended with the polyfunctional organic compound having two amide groups, represented by the general formula (1). This effectively suppresses such degradation under heating. Specifically, when the acrylic polymer composition is heated to 190°C or higher, the polyfunctional organic compound represented by the general formula (1) reacts with an ester group, a carboxy group, or a cleaved end, etc. contained in the acrylic polymer to form a new chemical bond between the polymer molecules. This appropriately suppresses such decrease in molecular weight. As a result, the thermal degradation under heating can be effectively suppressed. Specifically, in the acrylic polymer composition of one embodiment of the present invention, the polyfunctional organic compound represented by the general formula (1) functions as a repairing agent upon heating to 190°C or higher and can thereby effectively suppress thermal degradation under heating.

Particularly, the present inventors have found that by combined use of the acrylic polymer and the polyfunctional organic compound represented by the general formula (1), the polyfunctional organic compound represented by the general formula (1) effectively reacts against the molecular chain scission of the acrylic polymer at 190°C or higher, i.e., effectively functions as a repairing agent at 190°C or higher. Specifically, the polyfunctional organic compound represented by the general formula (1) reacts with an ester group, a carboxy group, or a cleaved end, etc. contained in the acrylic polymer at a temperature of 190°C or higher and does not effectively react as a repairing agent at a temperature of lower than 190°C, for example, 170°C or lower. Therefore, according to one embodiment of the present invention, the polyfunctional organic compound represented by the general formula (1) can appropriately suppress degradation under heating when the acrylic polymer composition is heated to 190°C or higher.

Specifically, percent decrease in molecular weight, specifically, peak top molecular weight (Mp), of the acrylic polymer contained in the acrylic polymer composition when the acrylic polymer composition of one embodiment of the

present invention is heated under conditions of 190°C and 144 hours in air (hereinafter, appropriately referred to as "percent decrease in molecular weight upon heating at 190°C") can be preferably controlled to less than 50%, more preferably 49.9% or less, further preferably 45.0% or less, still further preferably 40.0% or less.

The percent decrease in molecular weight upon heating at 190°C can be determined according to the following expression from the peak top molecular weight of the acrylic polymer contained in the acrylic polymer composition before heating, and the peak top molecular weight of the acrylic polymer contained in the acrylic polymer composition after the heating:

"Percent decrease in molecular weight upon heating at 190°C" (%) = 100 - ("Peak top molecular weight of the acrylic polymer after the heating" / "Peak top molecular weight of the acrylic polymer before the heating") × 100

The peak top molecular weights of the acrylic polymer before and after the heating can be measured, for example, as polystyrene-based values by gel permeation chromatography.

In the general formula (1), $R^1$ and $R^2$ are each independently a hydrogen atom, or an alkyl group having 1 to 20 carbon atoms, preferably a hydrogen atom. More preferably, both $R^1$ and $R^2$ are hydrogen atoms. When k is 2 or larger, pluralities of $R^1$ and $R^2$ are present. These pluralities of $R^1$ and $R^2$ may be the same as each other or may be different from each other and are preferably the same as each other. k is an integer of 1 or larger, preferably an integer of 2 to 300, more preferably an integer of 3 to 50, particularly preferably k = 6.

When $R^1$ or $R^2$ is an alkyl group having 1 to 20 carbon atoms, examples of the alkyl group having 1 to 20 carbon atoms include a methyl group, an ethyl group, a n-propyl group, an isopropyl group, a n-butyl group, an isobutyl group, a sec-butyl group, a t-butyl group, a n-pentyl group, a n-hexyl group, a n-heptyl group, a n-octyl group, a n-nonyl group, a n-decyl group, a cyclopropyl group, a cyclopentyl group, a cyclohexyl group, a cycloheptyl group, a cyclooctyl group, and the like.

In the general formula (1), $R^3$ and $R^4$ are each independently preferably an alkyl group having 1 to 20 carbon atoms and optionally having a substituent, or an aryl group having 6 to 10 carbon atoms and optionally having a substituent. For the compound represented by the general formula (1), more preferably, both $R^3$ and $R^4$ are alkyl groups having 1 to 20 carbon atoms and optionally having a substituent, or both $R^3$ and $R^4$ are aryl groups having 6 to 10 carbon atoms and optionally having a substituent. Particularly preferably, both $R^3$ and $R^4$ are aryl groups having 6 to 10 carbon atoms and optionally having a substituent from the viewpoint of a longer half-life at 190°C and therefore excellent stability, and furthermore, a much better function as a repairing agent in combined use with the acrylic polymer. When both $R^3$ and $R^4$ are alkyl groups having 1 to 20 carbon atoms and optionally having a substituent, $R^3$ and $R^4$ may be the same groups as each other or may be different groups from each other and are preferably the same groups from the viewpoint that the polyfunctional organic compound can more appropriately exert a function as a repairing agent. Likewise, when both $R^3$ and $R^4$ are aryl groups having 6 to 10 carbon atoms and optionally having a substituent, $R^3$ and $R^4$ may be the same groups as each other or may be different groups from each other and are preferably the same groups from the viewpoint that the polyfunctional organic compound can more appropriately exert a function as a repairing agent.

When $R^3$ or $R^4$ is an alkyl group having 1 to 20 carbon atoms, examples of the alkyl group having 1 to 20 carbon atoms include a methyl group, an ethyl group, a n-propyl group, an isopropyl group, a n-butyl group, an isobutyl group, a sec-butyl group, a t-butyl group, a n-pentyl group, a n-hexyl group, a n-heptyl group, a n-octyl group, a n-nonyl group, a n-decyl group, a cyclopropyl group, a cyclopentyl group, a cyclohexyl group, a cycloheptyl group, a cyclooctyl group, and the like. Among them, an alkyl group having 1 to 12 carbon atoms is preferred, and an alkyl group having 1 to 8 carbon atoms is more preferred. The alkyl group having 1 to 20 carbon atoms may have a substituent. Examples of the substituent include: halogen atoms such as a fluorine atom, a chlorine atom, and a bromine atom; alkoxy groups having 1 to 10 carbon atoms, such as a methoxy group, an ethoxy group, and an isopropoxy group; a nitro group; a cyano group; aryl groups having 6 to 10 carbon atoms and optionally having a substituent, such as a phenyl group, a 4-methylphenyl group, a 2-chlorophenyl group, a 1-naphthyl group, and a 2-naphthyl group; and the like. These substituents can be located at an arbitrary position.

When the alkyl group having 1 to 20 carbon atoms has a substituent, the number of carbon atoms in the alkyl group having 1 to 20 carbon atoms does not include the number of carbon atoms in such a substituent. Specifically, for the alkyl group having 1 to 20 carbon atoms, the number of carbon atoms excluding carbon atoms contained in the substituent can fall within the range of 1 to 20. When $R^3$ is, for example, a methoxyethyl group, the organic group has 2 carbon atoms. Specifically, in this case, the methoxy group is a substituent. Therefore, the number of carbon atoms

in the organic group excludes the number of carbon atoms in the substituent methoxy group.

When $R^3$ or $R^4$ is an aryl group having 6 to 10 carbon atoms, examples of the aryl group having 6 to 10 carbon atoms include a phenyl group, a 1-naphthyl group, a 2-naphthyl group, and the like. Among them, a phenyl group is preferred. Particularly preferably, both $R^3$ and $R^4$ are phenyl groups. The aryl group having 6 to 10 carbon atoms may have a substituent. Examples of the substituent include: halogen atoms such as a fluorine atom, a chlorine atom, and a bromine atom; alkoxy groups having 1 to 10 carbon atoms, such as a methoxy group, an ethoxy group, and an isopropoxy group; a nitro group; a cyano group; aryl groups having 6 to 10 carbons and optionally having a substituent, such as a phenyl group, a 4-methylphenyl group, a 2-chlorophenyl group, a 1-naphthyl group, and a 2-naphthyl group; alkyl groups having 1 to 20 carbon atoms, such as a methyl group, an ethyl group, a n-propyl group, an isopropyl group, a n-butyl group, an isobutyl group, a sec-butyl group, a t-butyl group, a n-pentyl group, a n-hexyl group, a n-heptyl group, a n-octyl group, a n-nonyl group, a n-decyl group, a cyclopropyl group, a cyclopentyl group, a cyclohexyl group, a cycloheptyl group, and a cyclooctyl group; and the like. These substituents can be located at an arbitrary position. When the aryl group having 6 to 10 carbon atoms has a substituent, the number of carbon atoms in the aryl group having 6 to 10 carbon atoms does not include the number of carbon atoms in such a substituent.

When both $R^3$ and $R^4$ are phenyl groups, the compound represented by the general formula (1) is preferably a compound represented by the following general formula (2):

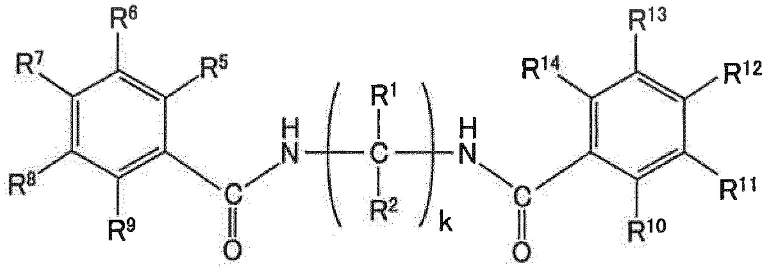

(2)

(in the general formula (2), $R^1$, $R^2$, and k are as defined in the general formula (1), and $R^5$ to $R^{14}$ are each independently a hydrogen atom, a halogen atom, an alkoxy group having 1 to 10 carbon atoms, a nitro group, a cyano group, a substituted or unsubstituted aryl group having 6 to 10 carbon atoms, or an alkyl group having 1 to 20 carbon atoms).

In the general formula (2), each of $R^5$ to $R^{14}$ is preferably a hydrogen atom, a cyano group, or an alkoxy group having 1 to 10 carbon atoms, more preferably a hydrogen atom or an alkoxy group having 1 to 10 carbon atoms. Also preferably, all of $R^5$ to $R^{14}$ are hydrogen atoms, or one of $R^5$ to $R^9$ is a cyano group or an alkoxy group having 1 to 10 carbon atoms, one of $R^{10}$ to $R^{14}$ is a cyano group or an alkoxy group having 1 to 10 carbon atoms, and the remining moieties are hydrogen atoms. Particularly preferably, among $R^5$ to $R^{14}$, each of $R^7$ and $R^{12}$ is a cyano group or an alkoxy group having 1 to 10 carbon atoms, and the remaining moieties are hydrogen atoms. The alkoxy group more preferably has 1 to 8 carbon atoms.

The polyfunctional organic compound represented by the general formula (1) is preferably a compound wherein both $R^1$ and $R^2$ are hydrogen atoms, $R^3$ is an alkyl group having 1 to 20 carbon atoms and optionally having a substituent, and $R^4$ is an alkyl group having 1 to 20 carbon atoms and optionally having a substituent; or a compound wherein both $R^1$ and $R^2$ are hydrogen atoms, $R^3$ is an aryl group having 6 to 10 carbon atoms and optionally having a substituent, and $R^4$ is an aryl group having 6 to 10 carbon atoms and optionally having a substituent, from the viewpoint of a much better function as a repairing agent in combined use with the acrylic polymer. A compound wherein both $R^1$ and $R^2$ are hydrogen atoms, $R^3$ is an aryl group having 6 to 10 carbon atoms and optionally having a substituent, and $R^4$ is an aryl group having 6 to 10 carbon atoms and optionally having a substituent is particularly preferred from the viewpoint of a much better function as a repairing agent in combined use with the acrylic polymer as well as a longer half-life at 190°C and therefore excellent stability.

The polyfunctional organic compound represented by the general formula (1), used in one embodiment of the present invention preferably has a molecular weight of 5,000 or smaller, more preferably a molecular weight of 4,500 or smaller, particularly preferably a molecular weight of 4,000 or smaller, from the viewpoint that the polyfunctional organic compound can more appropriately exert a function as a repairing agent. The lower limit of the molecular weight is not particularly limited and is usually 90 or larger.

The polyfunctional organic compound represented by the general formula (1), used in one embodiment of the present invention preferably has a half-life of 5 hours or longer, more preferably 8 hours or longer, further preferably 10 hours or longer, at 190°C from the viewpoint that the polyfunctional organic compound can exert a sufficient repairing function even when the acrylic polymer composition is heated to 190°C or higher. In one embodiment of the present invention, the half-life at 190°C can be defined as a value obtained by measuring decrease in weight of the polyfunctional organic compound represented by the general formula (1) by heating to 190°C, and determining a time at which the

weight becomes half the weight before the heating.

The content ratio of the polyfunctional organic compound represented by the general formula (1) in the acrylic polymer composition of one embodiment of the present invention is preferably an amount of 0.002 or more, more preferably an amount of 0.002 to 0.05, further preferably an amount of 0.0025 to 0.04, in terms of the number of equivalents of an amide group contained in the polyfunctional organic compound represented by the general formula (1) with respect to an ester group contained in the acrylic polymer. When the content ratio of the polyfunctional organic compound represented by the general formula (1) falls within the range described above, the degradation under heating of the acrylic polymer composition can be more appropriately prevented in the case of heating to a temperature of 190°C or higher. On the other hand, if the content of the polyfunctional organic compound represented by the general formula (1) is too small, the effect of suppressing the degradation under heating of the acrylic polymer composition may be insufficient in the case of heating to a temperature of 190°C or higher.

The content ratio of the polyfunctional organic compound represented by the general formula (1) in the acrylic polymer composition of one embodiment of the present invention can be an amount that allows the number of functional group equivalents to fall within the range described above. The weight ratio is preferably 0.4 parts by weight or more, more preferably 0.4 to 10 parts by weight, further preferably 0.5 to 8 parts by weight, per 100 parts by weight of the acrylic polymer.

<Additional component>

The acrylic polymer composition of one embodiment of the present invention preferably further comprises an antioxidant. Examples of the antioxidant that can be used include, but are not particularly limited to: aromatic secondary amine compounds such as phenyl-α-naphthylamine, octylated diphenylamine, 4,4'-bis(α,α-dimethylbenzyl)diphenylamine, p-(p-toluenesulfonylamido)diphenylamine, p-isopropoxy-diphenylamine, bis(phenyl-isopropylidene)-4,4-diphenylamine, N,N'-diphenyl-ethylenediamine, N,N'-diphenyl-propylenediamine, N,N'-diphenyl-p-phenylenediamine, N-isopropyl-N'-phenyl-p-phenylenediamine, N,N'-di-2-naphthyl-p-phenyldiamine,N-cyclohexyl-N'-phenyl-p-phenylenediamine, N-phenyl-N'-(3-methacryloyloxy-2-hydroxypropyl)-p-phenylenediamine, N,N'-bis(1-methylheptyl)-p-phenylenediamine, N,N-bis(1,4-dimethylpentyl)-p-phenylenediamine, 4-(α-phenylethyl)diphenylamine, 4,4'-bis(α-phenylethyl)diphenylamine, and 4,4'-bis(4-methylphenyl)sulfonyl)diphenylamine; and nickel dialkyldithiocarbamates such as nickel dimethyldithiocarbamate, nickel diethyldithiocarbamate, and nickel dibutyldithiocarbamate; and the like.

In one embodiment of the present invention, a compound represented by the following general formula (3a) or (3b) is more preferably used as the antioxidant from the viewpoint that degradation under heating can be more appropriately prevented upon heating to 190°C or higher:

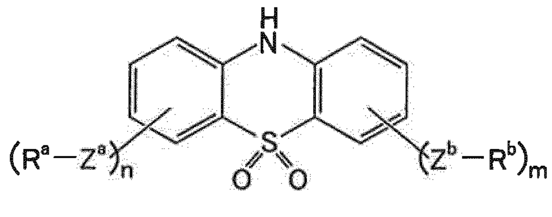

(3a)

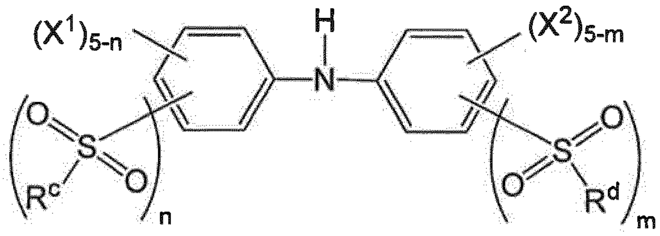

(3b)

(in the general formula (3a), $R^a$ and $R^b$ each independently represent an organic group having 1 to 30 carbon atoms and optionally having a substituent; $Z^a$ and $Z^b$ each independently represent a chemical single bond or $-SO_2-$; n and m are each independently 0 or 1, and at least one of n and m is 1. In the general formula (3b), $R^c$ and $R^d$ each independently represent an organic group having 1 to 30 carbon atoms and optionally having a substituent; $X^1$ and $X^2$ each independently represent a hydrogen atom, a halogen atom, and an alkyl group having 1 to 10 carbon atoms and optionally having a substituent, a cyano group, a nitro group, -OR, -O-C(=O)-R, -C(=O)-OR, -O-C(=O)-OR, NRR'-, -NR-C(=O)-R', -C(=O)-NRR', or -O-C(=O)-NRR' wherein R and R' each independently represent a hydrogen atom or an organic group having 1 to 20 carbon atoms and optionally having a substituent; all of a plurality of $X^1$ and a plurality of $X^2$ may be each

independently different substituents; n and m are each independently 0 or 1, and at least one of n and m is 1).

In the general formula (3a), $R^a$ and $R^b$ each independently represent an organic group having 1 to 30 carbon atoms and optionally having a substituent.

Examples of the organic group having 1 to 30 carbon atoms which constitutes $R^a$ or $R^b$ include, but are not particularly limited to: alkyl groups having 1 to 30 carbon atoms, such as a methyl group, an ethyl group, a n-propyl group, an isopropyl group, a n-butyl group, an isobutyl group, a sec-butyl group, a t-butyl group, a n-pentyl group, a n-hexyl group, a n-heptyl group, a n-octyl group, a n-nonyl group, and a n-decyl group; cycloalkyl groups having 3 to 30 carbon atoms, such as a cyclopropyl group, a cyclopentyl group, a cyclohexyl group, a cycloheptyl group, and a cyclooctyl group; aryl groups having 6 to 30 carbon atoms, such as a phenyl group, a biphenyl group, a naphthyl group, and an anthranyl group; alkoxy groups having 1 to 30 carbon atoms, such as a methoxy group, an ethoxy group, a n-propoxy group, an isopropoxy group, a n-butoxy group, an isobutoxy group, a sec-butoxy group, a t-butoxy group, a n-pentyloxy group, and a n-hexyloxy group; and the like.

The organic group mentioned above which constitutes $R^a$ or $R^b$ may have a substituent. The position of the substituent can be an arbitrary position.

When the organic group is an alkyl group, examples of such a substituent include: halogen atoms such as a fluorine atom, a chlorine atom, and a bromine atom; alkoxy groups having 1 to 10 carbon atoms, such as a methoxy group, an ethoxy group, and an isopropoxy group; a nitro group; a cyano group; phenyl groups optionally having a substituent, such as a phenyl group, a 4-methylphenyl group, and a 2-chlorophenyl group; and the like.

When the organic group is a cycloalkyl group or an aryl group, examples of the substituent include: halogen atoms such as a fluorine atom, a chlorine atom, and a bromine atom; alkoxy groups having 1 to 10 carbon atoms, such as a methoxy group, an ethoxy group, and an isopropoxy group; a nitro group; a cyano group; alkyl groups having 1 to 10 carbon atoms, such as a methyl group, an ethyl group, and a t-butyl group; and the like.

When the organic group is an alkoxy group, examples of the substituent include: halogen atoms such as a fluorine atom, a chlorine atom, and a bromine atom; a nitro group; a cyano group; and the like.

In the general formula (3a), when the organic group constituting $R^a$ or $R^b$ has a substituent, the number of carbon atoms in the organic group does not include the number of carbon atoms in the substituent.

$R^a$ and $R^b$ are each independently preferably an alkyl group having 2 to 20 carbon atoms and optionally having a substituent, or an aryl group having 6 to 30 carbon atoms and optionally having a substituent, more preferably a linear or branched alkyl group having 2 to 20 carbon atoms and optionally having a substituent, a phenyl group optionally having a substituent, or a naphthyl group optionally having a substituent, further preferably a linear or branched alkyl group having 2 to 8 carbon atoms and optionally having a substituent, or a phenyl group optionally having a substituent, particularly preferably a linear or branched alkyl group having 2 to 8 carbon atoms and optionally having a substituent. Examples of these substituents include the same as those listed as the substituents for the alkyl group having 1 to 30 carbon atoms and optionally having a substituent, and the aryl group having 6 to 30 carbon atoms and optionally having a substituent, as the organic group.

Preferred specific examples of such an organic group constituting $R^a$ or $R^b$ include an α-methylbenzyl group, an α,α-dimethylbenzyl group, a t-butyl group, a phenyl group, a 4-methylphenyl group, and the like. Among them, an α,α-dimethylbenzyl group or a 4-methylphenyl group is more preferred, and an α,α-dimethylbenzyl group is further preferred. These groups can be each independently selected.

In the general formula (3a), $Z^a$ and $Z^b$ are each independently a chemical single bond or $-SO_2-$, preferably a chemical single bond.

In the general formula (3a), n and m are each independently 0 or 1, and at least one of n and m is 1. Preferably, both n and m are 1.

In one embodiment of the present invention, the compound represented by the general formula (3a) is preferably any of compounds represented by the following general formulas (4) to (6):

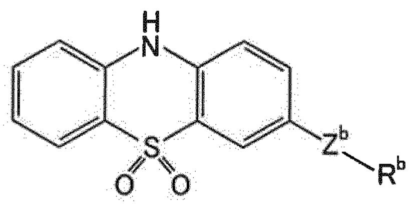
(4)

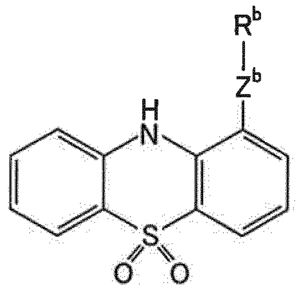
(5)

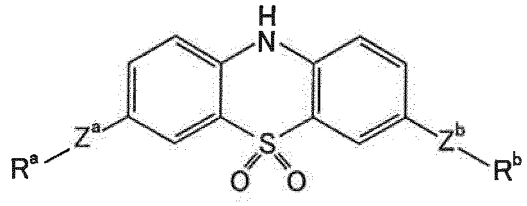

(6)

(in the general formulas (4) to (6), $R^a$, $R^b$, $Z^a$ and $Z^b$ are as defined in the general formula (3a)).

Among the compounds represented by the general formulas (4) to (6), the compound represented by the general formula (4) or (6) is preferred, and the compound represented by the general formula (6) is more preferred.

In the general formulas (4) to (6), $-Z^a-R^a$ and $-Z^b-R^b$ are each independently preferably an α-methylbenzyl group, an α,α-dimethylbenzyl group, a t-butyl group, a phenylsulfonyl group, or a 4-methylphenylsulfonyl group, more preferably an α,α-dimethylbenzyl group or a 4-methylphenylsulfonyl group, further preferably an α,α-dimethylbenzyl group.

Specifically, in one embodiment of the present invention, preferably, in the general formula (3a), $R^a$ and $R^b$ are each independently a linear or branched alkyl group having 2 to 8 carbon atoms and optionally having a substituent, each of $Z^a$ and $Z^b$ is a chemical single bond, and each of n and m is 1.

The compound represented by the general formula (3a) can be produced by obtaining a precursor phenothiazine compound by use of a known method for producing a phenothiazine compound, and subsequently oxidizing the obtained compound.

Specifically, the compound represented by the general formula (3a) can be obtained by using a compound represented by the following general formula (7) (phenothiazine) as a starting material, introducing substituents ($-Z^a-R^a$ and $-Z^b-R^b$) to positions 1, 3, 6 and/or 8 of the phenothiazine ring in the general formula (7) by a reaction method described in WO2011/093443A1, and oxidizing S of the phenothiazine ring into $-SO_2-$:

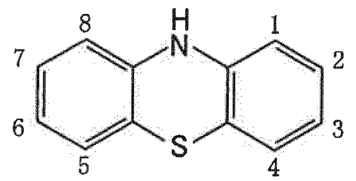

(7)

In the general formula (3b), $R^c$ and $R^d$ each independently represent an organic group having 1 to 30 carbon atoms and optionally having a substituent, and an aromatic group or a cyclic aliphatic group having 1 to 30 carbon atoms and optionally having a substituent is preferred.

Examples of the aromatic group having 1 to 30 carbon atoms include, but are not particularly limited to: aromatic hydrocarbon groups such as a phenyl group, a biphenyl group, a naphthyl group, a phenanthryl group, and an anthranyl group; and aromatic heterocyclic groups such as a furyl group, a pyrrolyl group, a thienyl group, a pyridyl group, and a thiazolyl group.

Examples of the cyclic aliphatic group having 1 to 30 carbon atoms include, but are not particularly limited to, a cyclopropyl group, a cyclobutyl group, a cyclopentyl group, a cyclohexyl group, and the like. Among them, $R^c$ and $R^d$ are each independently preferably a phenyl group or a 4-methylphenyl group.

The organic group mentioned above which constitutes $R^c$ or $R^d$ may have a substituent. The position of the substituent can be an arbitrary position. Examples of such a substituent include: halogen atoms such as a fluorine atom, a chlorine atom, and a bromine atom; alkoxy groups having 1 to 10 carbon atoms, such as a methoxy group, an ethoxy group, and an isopropoxy group; a nitro group; a cyano group; alkyl groups having 1 to 10 carbon atoms, such as a methyl group, an ethyl group, and a t-butyl group; and the like.

In the general formula (3b), when the organic group constituting $R^c$ or $R^d$ has a substituent, the number of carbon atoms in the organic group does not include the number of carbon atoms in the substituent.

In the general formula (3b), $X^1$ and $X^2$ each independently represent a hydrogen atom, a halogen atom, an alkyl group having 1 to 10 carbon atoms and optionally having a substituent, such as a methyl group, an ethyl group, a n-propyl group, an isopropyl group, a n-butyl group, an isobutyl group, a sec-butyl group, a t-butyl group, a n-pentyl group, a n-hexyl group, a n-heptyl group, a n-octyl group, a n-nonyl group, or a n-decyl group, a cyano group, a nitro group, -OR, -O-C(=O)-R, -C(=O)-OR, -O-C(=O)-OR, NRR'-, -NR-C(=O)-R', -C(=O)-NRR', or -O-C(=O)-NRR'. In this context, R and R' each independently represent a hydrogen atom or an organic group having 1 to 20 carbon atoms and optionally having a substituent. All of a plurality of $X^1$ and a plurality of $X^2$ may be each independently different substituents. All of $X^1$ and $X^2$ are preferably hydrogen atoms.

Examples of the substituent for the alkyl group having 1 to 10 carbon atoms and optionally having a substituent, represented by $X^1$ or $X^2$ include the same as those listed as the substituent for the alkyl group having 1 to 30 carbon atoms and optionally having a substituent, as $R^a$ or $R^b$.

In one embodiment of the present invention, the compound represented by the general formula (3b) is preferably selected as a compound wherein $R^c$ and $R^d$ each independently represent an aromatic group or a cyclic aliphatic group having 1 to 30 carbon atoms and optionally having a substituent, each of $X^1$ and $X^2$ represents a hydrogen atom, and each of n and m represents 1, more preferably a compound represented by the following general formula (3c):

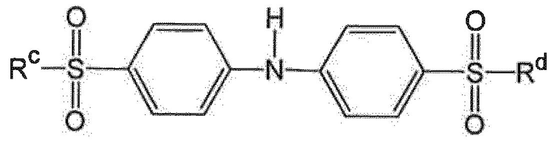

(3c)

(in the general formula (3c), $R^c$ and $R^d$ are as defined in the general formula (3b)).

The compound represented by the general formula (3b) can be produced by use of a known production method. The compound represented by the general formula (4b) can be synthesized by use of, for example, a reaction method described in WO2011/058918A1.

The content of the antioxidant in the acrylic polymer composition of one embodiment of the present invention is preferably 0.1 to 10 parts by weight, more preferably 0.3 to 5 parts by weight, further preferably 0.5 to 2.5 parts by weight, per 100 parts by weight of the acrylic polymer.

The acrylic polymer composition of one embodiment of the present invention may further comprise a cross-linking agent. The acrylic polymer composition of one embodiment of the present invention containing the cross-linking agent can be cross-linkable (cross-linkable acrylic polymer composition) and can be subjected to cross-linking reaction by heating or the like to prepare a rubber cross-linked product.

Examples of the cross-linking agent that can be used include, but are not particularly limited to, heretofore known cross-linking agents including: polyvalent amine compounds such as diamine compounds, and carbonates thereof; sulfur; sulfur donors; triazine thiol compounds; organic carboxylic acid ammonium salts; dithiocarbamic acid metal salts; polyvalent carboxylic acids; quaternary onium salts; imidazole compounds; isocyanuric acid compounds; organic peroxides; and the like. For example, the cross-linking agent can be appropriately selected according to the presence or absence of a cross-linkable monomer unit in the acrylic polymer or the type of the cross-linkable monomer unit. These cross-linking agents can be used alone or in combination of two or more thereof.

The polyvalent amine compound and the carbonate thereof are not particularly limited and are preferably a polyvalent amine compound having 4 to 30 carbon atoms, and a carbonate thereof. Examples of such a polyvalent amine compound and a carbonate thereof include aliphatic polyvalent amine compounds and carbonates thereof, aromatic polyvalent amine compounds, and the like. On the other hand, a compound having a non-conjugated nitrogen-carbon double bond, as in a guanidine compound, is not included therein.

Examples of the aliphatic polyvalent amine compound and the carbonate thereof include, but are not particularly limited to, hexamethylenediamine, hexamethylenediamine carbamate, N,N'-dicinnamylidene-1,6-hexanediamine, and the like. Among them, hexamethylenediamine carbamate is preferred.

Examples of the aromatic polyvalent amine compound include, but are not particularly limited to, 4,4'-methylenedianiline, p-phenylenediamine, m-phenylenediamine, 4,4'-diaminodiphenyl ether, 3,4'-diaminodiphenyl ether, 4,4'-(m-phenylenediisopropylidene)dianiline, 4,4'-(p-phenylenediisopropylidene)dianiline, 2,2'-bis[4-(4-aminophenoxy)phenyl]propane, 4,4'-diaminobenzanilide, 4,4'-bis(4-aminophenoxy)biphenyl, m-xylylenediamine, p-xylylenediamine, 1,3,5-benzenetriamine, and the like. Among them, 2,2'-bis[4-(4-aminophenoxy)phenyl]propane is preferred.

Examples of the sulfur donor include dipentamethylene thiuram hexasulfide, triethyl thiuram disulfide, and the like.

Examples of the triazine thiol compound include 1,3,5-triazine-2,4,6-trithiol, 6-anilino-1,3,5-triazine-2,4-dithiol, 6-dibutylamino-1,3,5-triazine-2,4-dithiol, 6-diallylamino-1,3,5-triazine-2,4-dithiol, 6-octylamino-1,3,5-triazine-2,4-dithiol, and the like. Among them, 1,3,5-triazine-2,4,6-trithiol is preferred.

Examples of the carboxylic acid ammonium salt include ammonium benzoate, ammonium adipate, and the like.

Examples of the dithiocarbamic acid metal salt include zinc dimethyldithiocarbamate, and the like.

Examples of the polyvalent carboxylic acid include tetradecanedioic acid, and the like.

Examples of the quaternary onium salt include cetyl trimethylammonium bromide, and the like.

Examples of the imidazole compound include 2-methylimidazole, and the like.

Examples of the isocyanuric acid compound include ammonium isocyanurate, and the like.

In the case of blending the cross-linking agent into the acrylic polymer composition of one embodiment of the present invention, the amount of the cross-linking agent blended is preferably 0.05 to 20 parts by weight, more preferably

0.1 to 15 parts by weight, further preferably 0.3 to 12 parts by weight, per 100 parts by weight of the acrylic polymer. When the content of the cross-linking agent falls within the range described above, cross-linking is sufficiently performed. In the case of preparing a rubber cross-linked product, the resulting rubber cross-linked product can be excellent in mechanical properties.

The acrylic polymer composition of one embodiment of the present invention can also comprise a compounding agent which is usually used in the field of rubber processing, in addition to each component described above. Examples of such a compounding agent include: reinforcing fillers such as carbon black and silica; non-reinforcing fillers such as calcium carbonate and clay; cross-linking accelerators; light stabilizers; plasticizers; processing aids; lubricants; pressure-sensitive adhesives; lubricating agents; flame retardants; antifungal agents; antistatic agents; colorants; silane coupling agents; cross-linking retardants; and the like. The amount of these compounding agents blended is not particularly limited without inhibiting the object and effects of one embodiment of the present invention. These compounding agents can be appropriately blended in an amount according to the purpose of blending.

<Method for preparing acrylic polymer composition>

Examples of the method for preparing the acrylic polymer composition of one embodiment of the present invention include, but are not particularly limited to, a method of mixing the acrylic polymer and the polyfunctional organic compound represented by the general formula (1) with various compounding agents to be added, if necessary.

Examples of the mixing method include, but are not particularly limited to, kneading methods using kneading machines such as rolls, intermixes, kneaders, Banbury mixers, or screw mixers, and the like. The mixing may be performed in a solvent.

In the case of blending the cross-linking agent, the acrylic polymer composition can be prepared by kneading components except for the cross-linking agent and a heat-labile coagent and the like using a mixing machine such as a Banbury mixer, a Brabender mixer, an intermix, or a kneader, transferring the mixture to a roll or the like, and adding thereto the cross-linking agent and the heat-labile coagent and the like, followed by secondary kneading.

In this way, the acrylic polymer composition of one embodiment of the present invention can be obtained. The acrylic polymer composition of one embodiment of the present invention comprises the acrylic polymer and the polyfunctional organic compound represented by the general formula (1). According to the acrylic polymer composition of one embodiment of the present invention containing the polyfunctional organic compound represented by the general formula (1), the polyfunctional organic compound represented by the general formula (1) functions as a repairing agent upon heating to 190°C or higher and can thereby effectively suppress degradation under heating when the acrylic polymer composition is heated at a temperature of 190°C or higher.

In the case of blending the cross-linking agent into the acrylic polymer composition of one embodiment of the present invention, the resultant can be cross-linked to obtain a rubber cross-linked product.

The rubber cross-linked product is produced by molding and cross-linking the acrylic polymer composition containing the cross-linking agent. Examples of the method for molding and cross-linking the acrylic polymer composition include, but are not particularly limited to: a method of extruding a cross-linkable rubber composition into a molded article using a single-screw or multi-screw extruder, followed by cross-linking by heating; a method of molding a composition in a mold using an injection molding machine, an extrusion blow molding machine, a transfer molding machine, a press molding machine, or the like, and cross-linking the composition by heating for molding at the same time with the molding; and the like. Among these methods, a method using an extruder or an injection molding machine is preferred, and a method using an extruder is particularly preferred. The molding and the cross-linking may be performed at the same time, or the cross-linking may be performed after the molding, without particular limitations. Either of the approaches can be selected according to a molding method, a vulcanization method, the size of a molded article, etc.

The molding temperature in molding and cross-linking the acrylic polymer composition is preferably 15 to 220°C, more preferably 20 to 200°C. Also, the cross-linking temperature is preferably 100°C or higher, more preferably 120°C to 250°C. The cross-linking time can be arbitrarily selected within the range of 1 minute to 5 hours. A method, such as electrothermal heating, steam heating, oven heating, UHF (ultrahigh frequency) heating, or hot-air heating, which is usually used in rubber cross-linking can be appropriately selected as a heating method.

Depending on the shape, size, etc. of the rubber cross-linked product, the inside may not be sufficiently cross-linked even if the surface is cross-linked. Therefore, secondary cross-linking may be further performed by heating. The heating temperature in performing the secondary cross-linking is preferably 100 to 220°C, more preferably 130 to 210°C, and the heating time is preferably 30 minutes to 10 hours, more preferably 1 to 5 hours.

Such a rubber cross-linked product is obtained using the acrylic polymer composition of one embodiment of the present invention mentioned above. Therefore, when the rubber cross-linked product is heated to 190°C or higher, thermal degradation under heating can be effectively suppressed, as in the acrylic polymer composition of one embodiment of the present invention mentioned above, by the repairing effect of the polyfunctional organic compound represented by the general formula (1) even if molecular chain scission occurs in the acrylic polymer after the cross-linking.

Hence, the rubber cross-linked product thus obtained is preferably used, by exploiting its properties, as various seals such as O-rings, packings, diaphragms, oil seals, shaft seals, bearing seals, mechanical seals, well head seals, seals for electric or electronic equipment, and seals for pneumatic equipment; various gaskets such as a cylinder head gasket which is mounted to the joint between a cylinder block and a cylinder head, a rocker cover gasket which is mounted to the joint between a rocker cover and a cylinder head, an oil pan gasket which is mounted to the joint between an oil pan and a cylinder block or a transmission case, a gasket for a fuel cell separator which is mounted to between a pair of housings sandwiching a unit cell equipped with a positive electrode, an electrolyte plate and a negative electrode, and a gasket for a top cover of a hard disk drive; various belts; various hoses such as fuel hoses, turbo air hoses, oil hoses, radiator hoses, heater hoses, water hoses, vacuum brake hoses, control hoses, air conditioner hoses, brake hoses, power steering hoses, air hoses, marine hoses, risers, and flow lines; various boots such as CVJ boots, propeller shaft boots, constant-velocity joint boots, and rack and pinion boots; attenuation material rubber components for cushioning materials, dynamic dampers, rubber couplings, air springs, vibration-proofing materials, etc.; and the like.

## EXAMPLES

Hereinafter, one embodiment of the present invention will be more specifically described with reference to Examples and Comparative Examples. In each example, the term "part" is based on weight unless otherwise specified.
Various physical properties were evaluated according to the following methods:

[Measurement of half-life of polyfunctional organic compound]

To measure the half-life of the polyfunctional organic compound, the temperature was raised according to the temperature increase program given below using a thermogravimetry-differential thermal analysis apparatus ("TG/DTA7200", manufactured by Seiko Instruments Inc. (SII)) so that the heating temperature by the apparatus was set to 180°C. Then, decrease in weight was measured when the sample temperature was stabilized at 190°C. The time at which the weight was halved was determined and regarded as the half-life of the polyfunctional organic compound.
Temperature increase program: 30°C → increase at 50°C/min → keep at 170°C for 3 minutes → increase at 10°C/min → keep at 180°C for 300 minutes

[Measurement of peak top molecular weight (Mp) of acrylic polymer]

The peak top molecular weight (Mp) of the acrylic polymer was measured as a polystyrene-based molecular weight by dissolving a film of the acrylic polymer composition in DMF and performing measurement by gel permeation chromatography (GPC). Specific measurement conditions were as given below. In this measurement, a molecular weight of 1,000 or smaller was judged as being derived from the polyfunctional organic compound and was thus not taken into consideration for the determination of the peak top molecular weight (Mp) of the acrylic polymer.

Instrument: High-performance liquid chromatograph HPC-8220GPC manufactured by Tosoh Corp.
Column: SupeR AWM-H manufactured by Tosoh Corp. (two columns placed in series)
Temperature: 40°C
Detector: RI-8220 manufactured by Tosoh Corp.
Eluent: DMF (containing 10 mmol/L lithium bromide)

[Percent decrease in molecular weight upon heating at 190°C]

A film of the acrylic polymer composition was heated at 190°C for 144 hours in air to obtain a heated film of the acrylic polymer composition. Then, the peak top molecular weight (Mp) of the acrylic polymer contained in the acrylic polymer composition was determined in the same way as above as to the heated film of the acrylic polymer composition. The percent decrease in molecular weight upon heating at 190°C (%) was calculated according to the following expression:

"Percent decrease in molecular weight upon heating at 190°C" (%) = 100 - ("Peak top molecular weight of the acrylic polymer after the heating" / "Peak top molecular weight of the acrylic polymer before the heating") × 100

[Synthesis Example 1: Synthesis of acrylic polymer]

A polymerization reactor equipped with a thermometer, a stirring apparatus, a nitrogen introduction tube and a pressure reducing apparatus was charged with 200 parts of water, 3 parts of sodium lauryl sulfate, and 100 parts of ethyl acrylate. Oxygen was thoroughly removed by repetitive deaeration under reduced pressure and nitrogen replacement. Then, 0.002 parts of sodium formaldehyde sulfoxylate and 0.005 parts of cumene hydroperoxide were added to the reactor. Emulsion polymerization reaction was started at room temperature under normal pressure. The reaction was continued until the rate of conversion in polymerization reached 95%. The polymerization was terminated by the addition of a polymerization terminator. Then, the obtained emulsion polymer solution was coagulated with an aqueous magnesium sulfate solution, washed with water, and dried to obtain a rubbery acrylic polymer (polyethyl acrylate).

[Synthesis Example 2: Synthesis of polyfunctional organic compound 1]

A 500 cc four-neck flask equipped with a dropping funnel was charged with 11.53 g of hexamethylenediamine, 200 cc of methylene chloride, and 30.75 g of triethylamine and cooled in ice, and 35.14 g of benzoyl chloride was added dropwise thereto through the dropping funnel with stirring. After the completion of dropwise addition, the mixture was stirred at room temperature for 3.5 hours. The solvent was replaced with THF, and precipitates were collected by filtration. Then, the obtained precipitates were washed three times with water and then dried to obtain 30.28 g of polyfunctional organic compound 1 represented by the formula (8) given below (compound represented by the general formula (2) wherein $R^1$ and $R^2$ = a hydrogen atom, $R^5$ to $R^{14}$ = a hydrogen atom, and k = 6, molecular weight: 324.42) at a yield of 94%. The half-life at 190°C of the obtained polyfunctional organic compound 1 was measured according to the method mentioned above and was consequently 1351.4 hours.

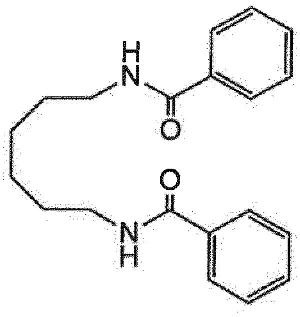

(8)

[Synthesis Example 3: Synthesis of polyfunctional organic compound 2]

A 500 cc four-neck flask equipped with a dropping funnel was charged with 11.56 g of hexamethylenediamine, 400 cc of THF, and 30.28 g of triethylamine and cooled in ice, and 42.53 g of 4-methoxybenzoyl chloride was added dropwise thereto through the dropping funnel with stirring. After the completion of dropwise addition, the mixture was stirred at room temperature for 1 hour, and precipitates were collected by filtration. Then, the obtained precipitates were washed three times with water and then dried to obtain 36.88 g of polyfunctional organic compound 2 represented by the formula (9) given below (compound represented by the general formula (2) wherein $R^1$ and $R^2$ = a hydrogen atom, $R^5$, $R^6$, $R^8$ to $R^{11}$, $R^{13}$, and $R^{14}$ = a hydrogen atom, $R^7$ and $R^{12}$ = a methoxy group, and k = 6, molecular weight: 384.47) at a yield of 96%. The half-life at 190°C of the obtained polyfunctional organic compound 2 was measured according to the method mentioned above and was consequently stable without decrease in weight at 190°C.

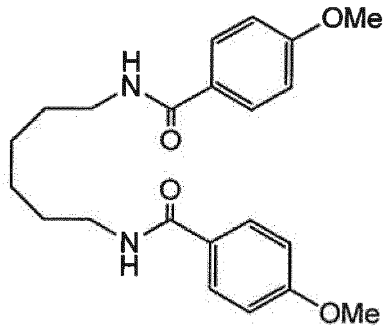

(9)

[Synthesis Example 4: Synthesis of polyfunctional organic compound 3]

A 500 cc four-neck flask equipped with a dropping funnel was charged with 4.58 g of hexamethylenediamine, 200 cc of THF, and 11.94 g of triethylamine and cooled in ice, and 14.29 g of 4-cyanobenzoyl chloride was added dropwise thereto through the dropping funnel with stirring. After the completion of dropwise addition, the mixture was stirred at room temperature for 2 hours, and precipitates were collected by filtration. Then, the obtained precipitates were washed three times with water and then dried to obtain 13.22 g of polyfunctional organic compound 2 represented by the formula (10) given below (compound represented by the general formula (2) wherein $R^1$ and $R^2$ = a hydrogen atom, $R^5$, $R^6$, $R^8$ to $R^{11}$, $R^{13}$, and $R^{14}$ = a hydrogen atom, $R^7$ and $R^{12}$ = a cyano group, and k = 6, molecular weight: 374.44) at a yield of 90%. The half-life at 190°C of the obtained polyfunctional organic compound 3 was measured according to the method mentioned above and was consequently 1724.1 hours.

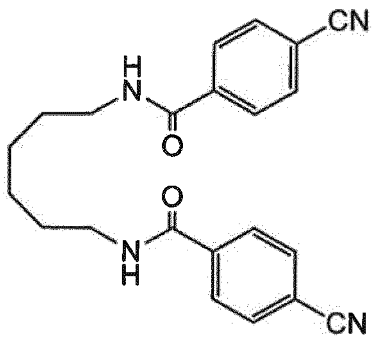

(10)

[Synthesis Example 5: Synthesis of polyfunctional organic compound 4]

A 500 cc four-neck flask equipped with a dropping funnel was charged with 9.33 g of hexamethylenediamine, 250 cc of THF, and 24.36 g of triethylamine and cooled in ice, and 23.61 g of hexanoyl chloride was added dropwise thereto through the dropping funnel with stirring. After the completion of dropwise addition, the mixture was stirred at room temperature for 1.6 hours, and precipitates were collected by filtration. Then, the obtained precipitates were washed three times with water and then dried to obtain 22.52 g of polyfunctional organic compound 2 represented by the formula (11) given below (compound represented by the general formula (1) wherein $R^1$ and $R^2$ = a hydrogen atom, $R^3$ and $R^4$ = a n-pentyl group, and k = 6, molecular weight: 312.49) at a yield of 90%. The half-life at 190°C of the obtained polyfunctional organic compound 3 was measured according to the method mentioned above and was consequently 78.4 hours.

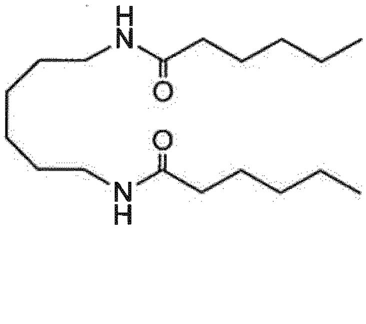

(11)

[Synthesis Example 6: Synthesis of antioxidant]

An antioxidant represented by the following formula (12) was synthesized according to the method given below:

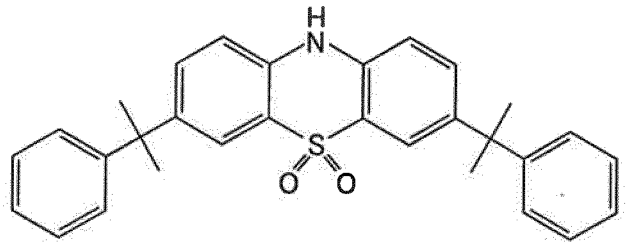

(12)

Specifically, first, 50.0 g (250.92 mmol) of phenothiazine was added to a three-neck reactor equipped with a thermometer in the stream of nitrogen and dissolved in 200 ml of toluene. Subsequently, 59.31 g (501.83 mmol) of α-methylstyrene and 1.19 g (6.27 mmol) of p-toluenesulfonic acid monohydrate were added to this solution and reacted at 80°C for 1 hour. Then, the reaction solution was brought back to room temperature, and 48 ml of acetic acid and 85.34 g (752.7 mmol) of a 30% aqueous hydrogen peroxide solution were added thereto and further reacted at 80°C for 2 hours. The reaction solution was brought back to room temperature and then added to 630 ml of methanol. Then, precipitated crystals were filtered and washed with 320 ml of methanol to obtain 85.7 g of an antioxidant represented by the formula (12) as white crystals at a yield of 73%.

[Example 1]

1 g of the acrylic polymer (polyethyl acrylate) obtained in Synthesis Example 1 was dissolved in 9 g of THF. To this solution, 23.1 mg of the antioxidant obtained in Synthesis Example 6 and 40.6 mg (0.125 mmol, the number of functional group equivalents with respect to an ester group contained in the acrylic polymer: 0.025) of the polyfunctional organic compound 1 obtained in Synthesis Example 2 were added, and the mixture was stirred overnight. 1.2 g of the obtained mixture was collected into a 6 cc sample bottle and dried under reduced pressure overnight at 40°C to obtain a film of an acrylic polymer composition. Then, the obtained film of the acrylic polymer composition was subjected to the measurement of the peak top molecular weight (Mp) of the acrylic polymer before heating and the measurement of the peak top molecular weight (Mp) of the acrylic polymer after heating at 190°C for 144 hours according to the method described above. As a result, the peak top molecular weight of the acrylic polymer was 820,344 before the heating and 588,466 after the heating for 144 hours, and the percent decrease in molecular weight upon heating at 190°C was 28.27%.

[Example 2]

A film of an acrylic polymer composition was obtained in the same way as in Example 1 except that the amount of the blended polyfunctional organic compound 1 obtained in Synthesis Example 2 was changed to 10.1 mg (0.0312 mmol, the number of functional group equivalents with respect to an ester group contained in the acrylic polymer: 0.0062). As a result of then conducting evaluation in the same way as in Example 1, the peak top molecular weight of the acrylic polymer was 810,814 before the heating and 456,222 after the heating for 144 hours, and the percent decrease in molecular weight upon heating at 190°C was 43.73%.

[Example 3]

A film of an acrylic polymer composition was obtained in the same way as in Example 1 except that the polyfunctional organic compound 2 obtained in Synthesis Example 3 was blended in an amount of 48.1 mg (0.125 mmol) instead of the polyfunctional organic compound 1 obtained in Synthesis Example 2. As a result of then conducting evaluation in the same way as in Example 1, the peak top molecular weight of the acrylic polymer was 829,973 before the heating and 655,465 after the heating for 144 hours, and the percent decrease in molecular weight upon heating at 190°C was 21.03%.

[Example 4]

A film of an acrylic polymer composition was obtained in the same way as in Example 3 except that the amount of the blended polyfunctional organic compound 2 obtained in Synthesis Example 3 was changed to 12.0 mg (0.0312 mmol) . As a result of then conducting evaluation in the same way as in Example 3, the peak top molecular weight of

the acrylic polymer was 764,633 before the heating and 540,820 after the heating for 144 hours, and the percent decrease in molecular weight upon heating at 190°C was 29.27%.

[Example 5]

A film of an acrylic polymer composition was obtained in the same way as in Example 1 except that the polyfunctional organic compound 3 obtained in Synthesis Example 4 was blended in an amount of 46.8 mg (0.125 mmol) instead of the polyfunctional organic compound 1 obtained in Synthesis Example 2. As a result of then conducting evaluation in the same way as in Example 1, the peak top molecular weight of the acrylic polymer was 792,051 before the heating and 527,886 after the heating for 144 hours, and the percent decrease in molecular weight upon heating at 190°C was 33.35%.

[Example 6]

A film of an acrylic polymer composition was obtained in the same way as in Example 5 except that the amount of the blended polyfunctional organic compound 3 obtained in Synthesis Example 4 was changed to 11.7 mg (0.0312 mmol) . As a result of then conducting evaluation in the same way as in Example 5, the peak top molecular weight of the acrylic polymer was 792,051 before the heating and 484,880 after the heating for 144 hours, and the percent decrease in molecular weight upon heating at 190°C was 38.78%.

[Example 7]

A film of an acrylic polymer composition was obtained in the same way as in Example 1 except that the polyfunctional organic compound 4 obtained in Synthesis Example 5 was blended in an amount of 39.1 mg (0.125 mmol) instead of the polyfunctional organic compound 1 obtained in Synthesis Example 2. As a result of then conducting evaluation in the same way as in Example 1, the peak top molecular weight of the acrylic polymer was 801,384 before the heating and 540,820 after the heating for 144 hours, and the percent decrease in molecular weight upon heating at 190°C was 32.51%.

[Comparative Example 1]

A film of an acrylic polymer composition was obtained in the same way as in Example 1 except that the polyfunctional organic compound 1 obtained in Synthesis Example 2 was not blended. As a result of then conducting evaluation in the same way as in Example 1, the peak top molecular weight of the acrylic polymer was 792,051 before the heating and 86,008 after the heating for 144 hours, and the percent decrease in molecular weight upon heating at 190°C was 89.14%.

[Comparative Example 2]

A film of an acrylic polymer composition was obtained in the same way as in Example 1 except that hexamethylenediamine carbamate (trade name "Diak #1", manufactured by DuPont Elastomer Co., Ltd.) was blended in an amount of 20.0 mg (0.125 mmol) instead of the polyfunctional organic compound 1 obtained in Synthesis Example 2. As a result of then conducting evaluation in the same way as in Example 1, the peak top molecular weight of the acrylic polymer was 810,814 before the heating and 187,754 after the heating for 144 hours, and the percent decrease in molecular weight upon heating at 190°C was 76.84%.

[Table 1]

Table 1

| | Polyfunctional organic compound | | | Percent decrease in molecular weight by heating at 190°C (%) |
|---|---|---|---|---|
| | Type | The number of functional group equivalent with respect to ester group in acrylic polymer | Amount blended per 1 g of acrylic polymer, (mmol) | |
| Example 1 | 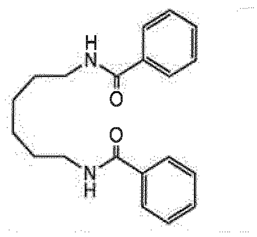 | 0.025 | 0.125 | 28.27 |
| Example 2 | | 0.0062 | 0.0312 | 43.73 |
| Example 3 | 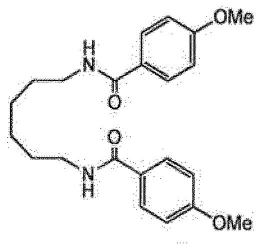 | 0.025 | 0.125 | 21.03 |
| Example 4 | | 0.0062 | 0.0312 | 29.27 |
| Example 5 | 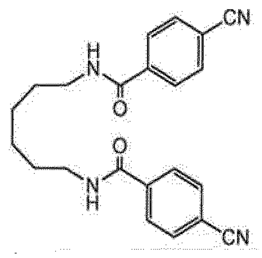 | 0.025 | 0.125 | 33.35 |
| Example 6 | | 0.0062 | 0.0312 | 38.78 |
| Example 7 | 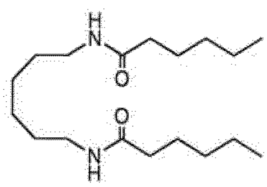 | 0.025 | 0.125 | 32.51 |
| Com parative Example 1 | None | - | - | 89.14 |
| Comparative Example 2 | Hexamethylenediamine carbamate | 0.025 | 0.125 | 76.84 |

As shown in Table 1, according to Examples 1 to 7, an acrylic polymer composition was able to be obtained which contained an acrylic polymer blended with a polyfunctional organic compound represented by the general formula (1), and was controlled to have less than 50% percent decrease in molecular weight upon heating at 190°C. Furthermore, such an acrylic polymer composition can effectively suppress decrease in molecular weight of the acrylic polymer caused by molecular chain scission even upon heating to 190°C or higher and can therefore be appropriately prevented from being thermally degraded under heating. As for such an effect, a similar effect can be obtained even when the acrylic polymer composition is supplemented with a cross-linking agent and the like and prepared into a cross-linked product.

By contrast, both in Comparative Example 1 in which the polyfunctional organic compound represented by the general formula (1) was not blended, and in Comparative Example 2 in which hexamethylenediamine carbamate was blended instead of the polyfunctional organic compound represented by the general formula (1), the percent decrease in molecular weight upon heating at 190°C exceeded 50%, resulting in marked decrease in molecular weight upon

heating to 190°C or higher.

## Claims

1. An acrylic polymer composition comprising an acrylic polymer and a polyfunctional organic compound represented by the following general formula (1):

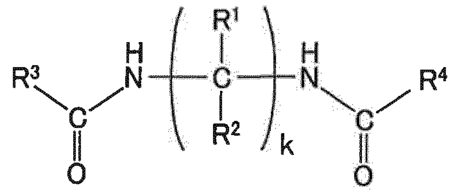

(1)

(in the general formula (1), $R^1$ and $R^2$ are each independently a hydrogen atom or an alkyl group having 1 to 20 carbon atoms, $R^3$ and $R^4$ are each independently a hydrogen atom, a substituted or unsubstituted alkyl group having 1 to 20 carbon atoms, or a substituted or unsubstituted aryl group having 6 to 10 carbon atoms, and k is an integer of 1 or larger).

2. The acrylic polymer composition according to claim 1, wherein the polyfunctional organic compound is a compound represented by the following general formula (2):

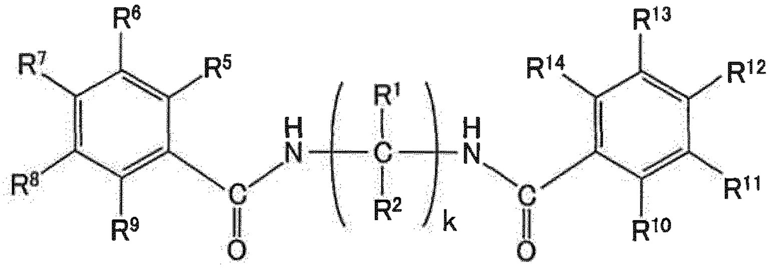

(2)

(in the general formula (2), $R^1$, $R^2$, and k are as defined in the general formula (1), and $R^5$ to $R^{14}$ are each independently a hydrogen atom, a halogen atom, an alkoxy group having 1 to 10 carbon atoms, a nitro group, a cyano group, a substituted or unsubstituted aryl group having 6 to 10 carbon atoms, or an alkyl group having 1 to 20 carbon atoms).

3. The acrylic polymer composition according to claim 1 or 2, wherein k is 6.

4. The acrylic polymer composition according to any of claims 1 to 3, wherein the polyfunctional organic compound has a half-life of 5 hours or longer at 190°C.

5. The acrylic polymer composition according to any of claims 1 to 4, wherein a content ratio of the polyfunctional organic compound is an amount of 0.002 or more in terms of the number of equivalents of the amide group contained in the polyfunctional organic compound with respect to an ester group contained in the acrylic polymer.

6. The acrylic polymer composition according to any of claims 1 to 5, wherein a content ratio of the polyfunctional organic compound is 0.4 parts by weight or more in terms of a weight ratio per 100 parts by weight of the acrylic polymer.

7. A cross-linked product prepared by cross-linking an acrylic polymer composition according to any of claims 1 to 6.

8. A cross-linkable acrylic polymer composition comprising an acrylic polymer, a polyfunctional organic compound represented by the following general formula (1), and a cross-linking agent:

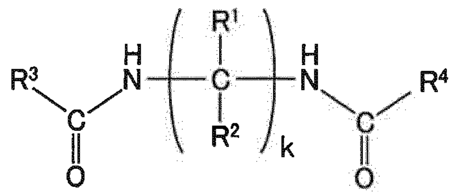

(1)

(in the general formula (1), $R^1$ and $R^2$ are each independently a hydrogen atom or an alkyl group having 1 to 20 carbon atoms, $R^3$ and $R^4$ are each independently a hydrogen atom, a substituted or unsubstituted alkyl group having 1 to 20 carbon atoms, or a substituted or unsubstituted aryl group having 6 to 10 carbon atoms, and k is an integer of 1 or larger).

## INTERNATIONAL SEARCH REPORT

International application No. PCT/JP2017/011459

**A. CLASSIFICATION OF SUBJECT MATTER**

*C08L33/00*(2006.01)i, *C08K5/20*(2006.01)i, *C07C233/36*(2006.01)n, *C07C233/78*(2006.01)n, *C07C235/50*(2006.01)n, *C07C255/57*(2006.01)n

According to International Patent Classification (IPC) or to both national classification and IPC

**B. FIELDS SEARCHED**

Minimum documentation searched (classification system followed by classification symbols)
C07B31/00-61/00, 63/00-63/04, C07C1/00-409/44, C08K3/00-13/08, C08L1/00-101/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 4-28748 A (Sekisui Chemical Co., Ltd.), 31 January 1992 (31.01.1992), claims; page 1, right column, lines 13 to 18; examples 5, 6, 7, 10 (Family: none) | 1,3-8<br>2 |
| Y<br>A | JP 60-240735 A (Mitsubishi Petrochemical Co., Ltd.), 29 November 1985 (29.11.1985), page 2, lower right column, lines 7 to 13; examples 14, 15, 20 & US 4663375 A1 claims; column 3, lines 8 to 21; examples 14, 15, 20 & EP 162663 A2 | 2<br>1,3-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 April 2017 (21.04.17) | 09 May 2017 (09.05.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No.
PCT/JP2017/011459

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2001-309830 A (Okamoto Industries, Inc.), 06 November 2001 (06.11.2001), claims; blending composition A; example 1 (Family: none) | 1,4-8<br>2,3 |
| X<br>A | JP 2001-328395 A (Okamoto Industries, Inc.), 27 November 2001 (27.11.2001), claims; blending composition C; example 1 (Family: none) | 1,4-8<br>2,3 |
| X<br>A | WO 2011/152449 A1 (Kaneka Corp.), 08 December 2011 (08.12.2011), claims; example 11 & US 2013/0183536 A1 paragraph [0230]; Experimental example 45 | 1,4-8<br>2,3 |
| X<br>A | JP 55-78048 A (Nippon Kasei Chemical Co., Ltd.), 12 June 1980 (12.06.1980), claims; page 3, upper left column, lines 1 to 3; example 6 (Family: none) | 1,3,4,7,8<br>2,5,6, |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5682575 B
- JP 2005023166 A
- WO 2011093443 A1
- WO 2011058918 A1